# EUROPEAN PATENT APPLICATION

(11) **EP 4 763 107 A1**
(43) Date of publication of application: **24.06.2026**
(21) Application number: 24944172.6
(22) Date of filing: 30.08.2024
(51) Int. Cl.: A61B 17/22

(54) **INTRODUCER SHEATH AND PERFUSION AND SUCTION SYSTEM**

(30) Priority: 28.06.2024 CN 202410859650
(71) Applicant: Zhejiang Yigao Medical Technology Co., Ltd., Hangzhou, Zhejiang 311422 (CN)
(72) Inventor: LI, Fangbing, Hangzhou, Zhejiang 311422 (CN); LI, Xing, Hangzhou, Zhejiang 311422 (CN); LU, Sundie, Hangzhou, Zhejiang 311422 (CN)
(74) Representative: Cabinet Chaillot
(86) International application number: PCT/CN2024/115815
(87) International publication number: WO 2026/000598

(57) **Abstract**

A perfusion and suction system and an introducer sheath are provided. The introducer sheath comprises a sheath tube and a handle. The handle is connected to a proximal side of the sheath tube so that the handle can be kept outside the body; the handle body extends in a direction consistent with the axis of the sheath tube for easy holding by a user; the handle body defines a port communicated with a first lumen so that medical equipment can enter the first lumen through the port; the first connecting member has a first channel, the first channel is communicated with the first lumen to form a drainage path, the first connecting member is suitable for connecting with a negative pressure device, and the pressure regulating member is arranged away from the drainage path so as not to contact the liquid when adjusting pressure of the drainage path.

## Description

### CROSS-REFERENCE TO RELATED APPLICATIONS

### TECHNICAL FIELD

The present disclosure relates to a perfusion and suction system and an introducer sheath.

### BACKGROUND

In a conventional ureteral lithotripsy with flexible endoscopy, the powder of stones and hematuria in renal pelvis can lead to blurred vision, which requires perfusion solution to maintain clear vision. However, at the same time, the intrapelvic pressure will increase significantly due to rapid perfusion and poor return, causing infected urine, bacteria and endotoxin to enter the blood and lymphatic circulation, resulting in postoperative fever, systemic inflammatory response syndrome and even fatal urogenous sepsis in patients. In order to prevent severe infection caused by excessive pressure in the renal pelvis during a flexible endoscopy surgery, it is necessary to control the intraoperative pressure within a safe range. It is further necessary to feedback and adjust the perfusion flow rate and/or negative suction value according to the intraoperative pressure of the renal pelvis. Whether the manometric method used can measure the pressure of the renal pelvis in real time and accurately is the cornerstone to ensure the good performance of the system and the safety of surgical operation.

A pressure-measuring ureteral access sheath 10 is mainly used clinically to establish a surgical channel when a relevant surgery is required for treatment of ureteral stenosis, adhesion, obstruction, blockage, and calculus. The endoscope 20, laser fiber, stone removal instrument or operating cable and so on are introduced through the channel. Moreover, in existing urological surgeries, perfusion solution is required to maintain clear vision. However, the intrapelvic pressure will increase significantly due to rapid perfusion and poor return. To prevent a series of complications such as fever caused by changes in intrapelvic pressure and renal pelvic rupture, it is necessary to control the intrapelvic pressure within a safe range.

The existing bendable pressure measuring sheath has the pressure measuring lumen retained, and the sensor and the pressure measuring control module are placed on the main controller or the main controller tubeline, and then connected to the pressure measuring lumen of the pressure measuring sheath by means of the connecting tubeline, thereby transmitting pressure to the sensor. However, the pressure measuring precision of this structure is greatly affected by the tubeline, and the pressure measuring precision is poor. In addition, the operation buttons are located on the host controller, which is not convenient for doctors to control and operate, and it is difficult to control a perfusion and suction process while focusing on crushing and removing stones. In addition, there are some solutions in which the pressure sensor is mounted on a pressure measuring sheath or an endoscope, and the pressure measuring control module is connected to the host controller or the host controller tubeline by means of a cable. This structure has poor control operability and the pressure measuring precision is not completely guaranteed.

### SUMMARY

The present disclosure provides an introducer sheath and the introducer sheath comprises
a sheath tube, having a size and a shape suitable for passing through the lumen of a body into a target area in the body and extending longitudinally from a proximal end to a distal end, and the sheath tube defining at least a first lumen extending longitudinally from the proximal end to the distal end;
a handle, being connected to a proximal side of the sheath tube so that the handle can be kept outside the body, where the handle comprises a handle body and a first connecting member and a pressure regulating member arranged on the handle body; the handle body extends in a direction consistent with the axis of the sheath tube for easy holding by a user; the handle body defines a port communicated with a first lumen so that medical equipment can enter the first lumen through the port; the first connecting member has a first channel, the first channel is communicated with the first lumen to form a drainage path, the first connecting member is suitable for connecting with a negative pressure device so that liquid medium in the body can be discharged from the body through the drainage path, and the pressure regulating member is in fluid communication with the first lumen and is arranged away from the drainage path so as not to contact the liquid when adjusting pressure of the drainage path; and
a seal, being arranged at the port, where the seal comprises a passage passing therethrough so that after the medical equipment passes through the passage, a sealed cavity is formed between the first lumen and the body.

In some embodiments, the introducer sheath further comprises
a printed circuit board (PCB), being mounted in the handle body, where the PCB is provided with at least one data connector, the data connector is configured to be suitable for connecting an external perfusion and suction device to transmit signals; and
an operating button, being arranged on the handle body and connected to the PCB to control operating states of the perfusion and suction device.

In some embodiments, a pressure measuring lumen extending longitudinally from a proximal end to a distal end is formed in the wall of the sheath tube, a pressure measuring port for sensing the liquid pressure in the body is provided at the distal side of the pressure measuring lumen, a pressure sensor is provided on the pressure measuring lumen or any path connected thereto, and the pressure sensor is connected to the PCB; preferably, the handle comprises a second connecting member arranged on the handle body, the second connecting member has a second channel communicated with the pressure measuring lumen, and the pressure sensor is arranged at the second channel to sense the pressure in the second channel.

In some embodiments, a liquid outlet is provided at the bottom end of the first lumen, the first connecting member is located below the handle body, and the axis of the first channel is perpendicular to the axis of the first lumen to form a linear drainage path.

In some embodiments, a hand-held positioning portion is provided at the bottom of the handle body, the first connecting member is arranged at the bottom of the handle body, the second connecting member is arranged on a side wall of the handle, and the pressure regulating member and the operating button are located at the upper part of the handle.

In some embodiments, the handle comprises a third connecting member arranged on the handle body, the sheath tube defines an auxiliary lumen extending from the proximal end toward the distal end, and the third connecting member is communicated with the auxiliary lumen to form a fluid replenishment channel.

In some embodiments, the sheath tube is connected to the handle body in a sealed manner to construct a calibration cavity in the handle body, and the calibration cavity is connected to the outside world; a PCB and a pressure sensor are installed in the calibration cavity, the pressure sensor is a gauge pressure sensor, a pressure measuring hole communicated with the calibration cavity is provided on the outer wall of the second channel, the gauge pressure sensor is positioned in the cavity and at least a sensing film thereof is arranged at the pressure measuring hole to collect pressure in the pressure measuring lumen, and the second channel has an opening that can be connected to the outside world; the opening can be opened or closed by a sealing part, and the calibration of the pressure sensor is achieved by cooperating with the calibration cavity through the opening.

In some embodiments, the pressure regulating member comprises a relief valve arranged on the handle body, the handle body is provided with a relief cavity communicated with the first lumen, and the relief valve seals or opens the relief cavity; preferably, the relief valve comprises a pressing part, the pressing part is connected to a relief hose, and an openable and closable valve is formed on the relief hose.

In some embodiments, the second connecting member is configured to be suitable for connection with an external air intake device to eliminate water film tension of the pressure measuring lumen.

In some embodiments, an exhaust pipe communicating with the outside world and the calibration cavity is provided in the data connector to maintain pressure stability in the handle body.

In some embodiments, the seal is connected to the proximal end of the sheath tube, a pressing piece is provided at the proximal end of the handle body, and the pressing piece comprises a pressing part pressed above the seal and a rotating part that rotates with the handle body; the seal is provided with a lower pressure relief hole, and the pressing part is provided with an upper pressure relief hole; the pressing part is driven to rotate by the rotation of the rotating part so that the lower pressure relief hole is communicated with the upper pressure relief hole to achieve pressure stabilization.

In some embodiments, the pressure measuring port is communicated with the outside world and/or the first lumen; preferably, the distal end of the pressure measuring lumen is located at the proximal side to the distal end of the first lumen, and the distal end of the pressure measuring lumen is provided with a pressure measuring port that penetrates the outside world and the first lumen.

In some embodiments, a pressure sensor is provided at the distal side of the pressure measuring lumen, and a signal line is arranged in the pressure measuring lumen to transmit pressure signals collected by the pressure sensor to the PCB.

In some embodiments, the surface of at least the cavity at the distal side of the pressure measuring lumen is coated with a hydrophilic or hydrophobic coating.

In some embodiments, a one-way valve is provided on the second connecting member to allow only gas to enter the pressure measuring lumen from the outside world in one direction.

Another purpose of the present disclosure is to provide a perfusion and suction system. The system comprises an introducer sheath as described previously, an endoscope, being sealed through a port of the handle body into the first lumen, and waste liquid being discharged from a gap between the first lumen and the endoscope; a perfusion device, being connected to an endoscope to deliver a liquid medium into a human body through an endoscopic catheter; a suction device, being connected to the first connecting member to generate negative pressure in a drainage path so as to suck out waste liquid containing stones from the human body; and a host controller, the host controller being connected to the perfusion device, the suction device, and the PCB to control an operating status of perfusion and suction.

The present disclosure optimizes the performance of the perfusion and suction system through precise pressure monitoring and regulation, efficient discharge path, convenient pressure relief mechanism, multifunctional integrated handle design, efficient perfusion and suction system, and precision and response speed, and improves stone removal efficiency, pressure measurement precision and operation convenience.

Additional aspects and advantages of the present disclosure will be set forth in part in the following description, part of which will become apparent from the following description or as will be appreciated by practice of the present disclosure.

### BRIEF DESCRIPTION OF DRAWINGS

The above and/or additional aspects and advantages of the present disclosure will become apparent and readily understood from a description of embodiments in connection with the following drawings, in which:
FIG. 1 is a diagram illustrating a structure of a perfusion system involved in a pressure measuring sheath provided by the present disclosure;
FIG. 2 is a diagram illustrating a principle of a pressure measuring sheath provided by an embodiment of the present disclosure;
FIG. 3 is a diagram illustrating a structure of a pressure measuring sheath (with an expander) provided by the present disclosure;
FIG. 4 is a diagram illustrating a partial structure of a pressure measuring sheath (with an expander) of the present disclosure;
FIG. 5 is a diagram of a structural disassembly of a pressure measuring sheath (with an expander) provided by the present disclosure;
FIG. 6 is a diagram illustrating a structural section of a pressure measuring sheath provided by the present disclosure;
FIG. 7 is a diagram of a side view illustrating a structure of a pressure measuring sheath provided by the present disclosure;
FIG. 8 is a diagram illustrating a structural section of a pressure measuring sheath provided by the present disclosure;
FIG. 9 is a diagram illustrating a distal end of a pressure measuring sheath (with an expander) provided by the present disclosure;
FIGS.10-13 are diagrams illustrating structures of pressure measuring ports provided by the present disclosure;
FIG. 14 is a diagram of a pressure measuring sheath provided by the present disclosure with carbon dioxide pumped;
FIGS. 15-16 are schematic diagrams of the pressure detection principle of the pressure measuring sheath provided by the present disclosure.
FIGS. 17-18 are diagrams illustrating structural sections of distal ends of pressure measuring sheaths provided by the present disclosure;
FIG. 19 is a diagram illustrating a structural section of a cable provided by the present disclosure;
FIG. 20 is a diagram illustrating a structure of a sheath tube (with a sensor configured at the distal end) provided by the present disclosure; and
FIGS. 21-22 are diagrams illustrating operations of pressure measuring sheaths provided by the present disclosure.

### Reference numerals:

10-pressure measuring sheath, 11-data connector, 20-endoscope, and 200-endoscope catheter
101-handle body, 102-sheath tube, 103-calibration cavity, 104-first lumen, 105-pressure measuring lumen, 106-seal, 107-pressure measuring port, 108-port, 109-finger ring, 120-first connecting member, 121-third connecting member, 122-first sealing cap, 123-auxiliary lumen, 124-second connecting member, 125-second sealing cap, 126-pressure measuring hole, 127-pressure relief cavity, 18-pressure regulating member, 128-pressing part, 130-pressure relief hole, 131-switch, 132-first connecting part, 133-pressing part, 134-rotating part, 135-limiting part, 136-flexible section, and 138-valve;
50-pressure sensor, 51-PCB, 52-data connector, 521-wire core, 522-insulation sheath, 53- connector, 54-operating button, and 55-exhaust pipe;
60-expander, 601-second connection part, 602-expander cavity, 605-groove, 603-side hole, 604-plugging part, 70-connecting pipe, 71-carbon dioxide air pump, 73-hydrophilic or hydrophobic coating, and 74-one-way valve;
80-resistance bridge sensor, 81-stainless steel sleeve, and 82-signal line; and
90-first negative pressure suction tube, 91-suction container, 92-second negative pressure suction tube, 93-liquid storage container, 94-liquid inlet pipe, 95-liquid outlet pipe, 100-chassis, 201-endoscope wiring harness, and 202-image processor.

### DETAILED DESCRIPTION OF THE EMBODIMENTS

It should be understood in the description of the present disclosure that the directions or position relationships such as "front", "rear", "head", "tail", "distal", "proximal", "axial" and "radial" are based on directions or position relationships shown in the accompanying drawings, and are used only for facilitating the description of the present disclosure and simplifying the description, not for indicating or implying that the target devices or elements must have a special direction and be constructed and operated at the special direction, so they cannot be understood as the restrictions to the present disclosure.

It should be noted that in the description of the present disclosure that the terms "mount" and "connect" should be understood in a broad sense unless otherwise specified and defined. For example, it may refer to mechanical connection or electrical connection, and may also refer to internal connection or direct connection of two elements or indirect connection of two elements by an intermedium. The person of ordinary skill in the art can understand the specific meaning of the above terms according to specific conditions.

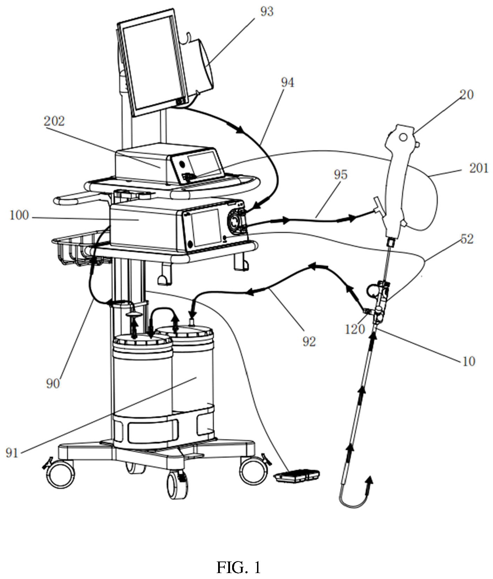

As shown in FIG. 1, an embodiment of the present disclosure provides a perfusion and suction system. The system comprises a pressure measuring sheath 10, an endoscope 20, a perfusion device, a suction device, a pressure detection component, and a host controller. The endoscope 20 is inserted into the pressure measuring sheath 10 to observe the renal pelvis; the perfusion device uses a liquid medium to pressurize and expand a patient's inner cavity to form a visible area, and can clean impurities in the cavity, so that the doctor can observe clearly through the endoscope 20 and have a clear surgical field of view; and the suction device is used to suck the waste liquid in the patient's inner cavity out of the body. The pressure detection component comprises a pressure sensor 50. The pressure sensor 50 is used to detect the intracavitary pressure in the renal pelvis. The pressure detection component, the perfusion device, and the suction device are connected to the host controller, that is, the host controller controls the flow rate and pressure of the perfusion device and the suction device according to the pressure value output by the pressure sensor 50. Specifically, the perfusion device delivers liquid into the cavity through the channel of the endoscope 20, and the suction device is used to negatively extract waste liquid and stones in the cavity through the channel of the pressure measuring sheath 10, collect the waste liquid and stones into the suction container 91 through the negative pressure suction tube, and perfusion device cooperates with the suction device to keep the cavity at a suitable pressure. Exemplarily, the suction device comprises a diaphragm pump, a first negative pressure suction tube 90, a suction container 91 and a second negative pressure suction tube 92, in which the first negative pressure suction tube 90 connects the diaphragm pump with the suction container 91, and the second negative pressure suction tube 92 connects the suction container 91 with the liquid outlet of the pressure measuring sheath 10. The perfusion device comprises a liquid storage container 93 and a perfusion pump. The perfusion pump is connected with the liquid storage bag through the liquid inlet pipe 94. The perfusion pump is communicated with a liquid delivery channel through a liquid outlet pipe 95. It can be understood that the liquid medium can enter the renal pelvis through the endoscope 20 and can also enter the renal pelvis via the pressure measuring sheath 10. Optionally, the perfusion pump, suction pump and host controller are integrated and installed on the same chassis 100. Specifically, the perfusion pump is a peristaltic pump and the suction pump is a diaphragm pump. In addition, the endoscope is connected to the image processor 202 through the endoscope wiring harness 201, and the host controller can be installed on the endoscope 20 or on the image processor 202.

The following embodiments are all based on existing perfusion suction equipment. The existing perfusion suction equipment aims to improve stone removal efficiency, pressure measurement precision, and convenient operation by improving the sheath tube 102, endoscope 20, expander 60, and perfusion suction equipment.

### Embodiment 1

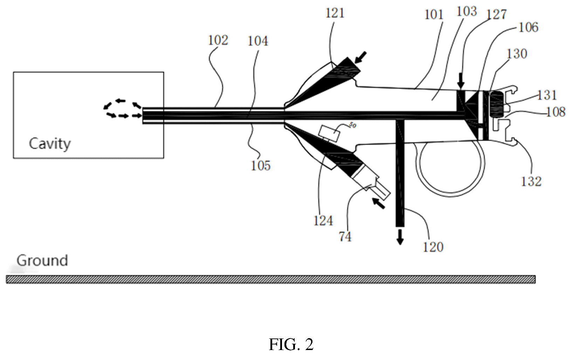

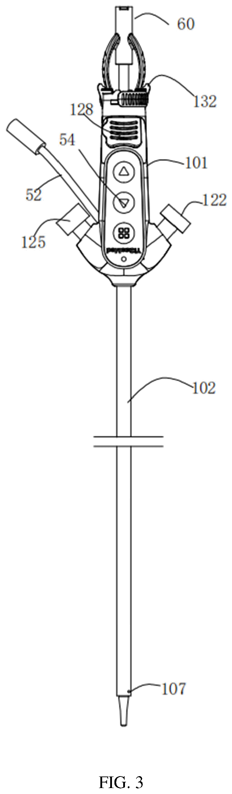

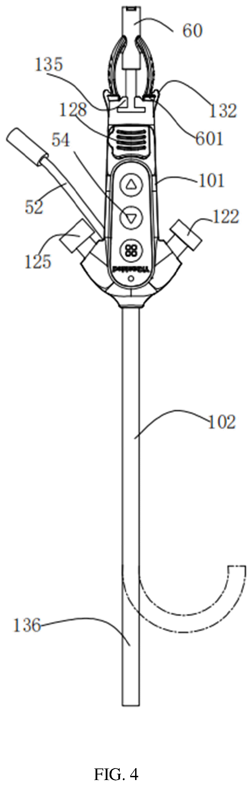

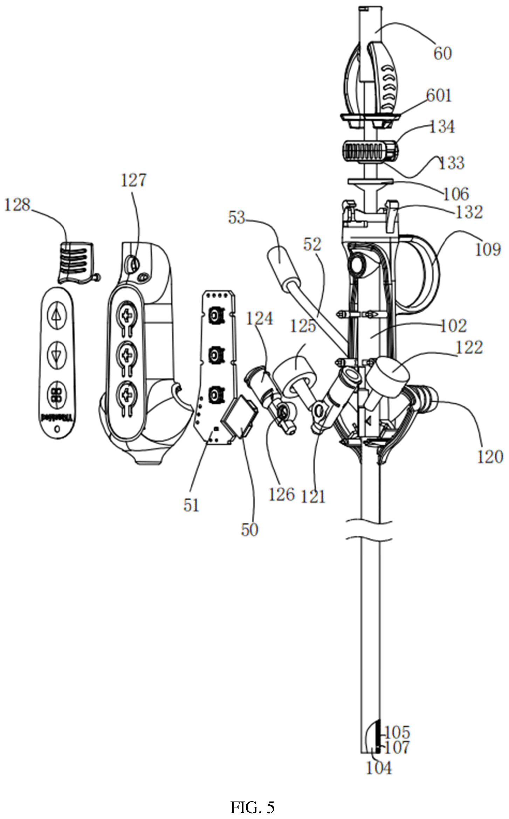

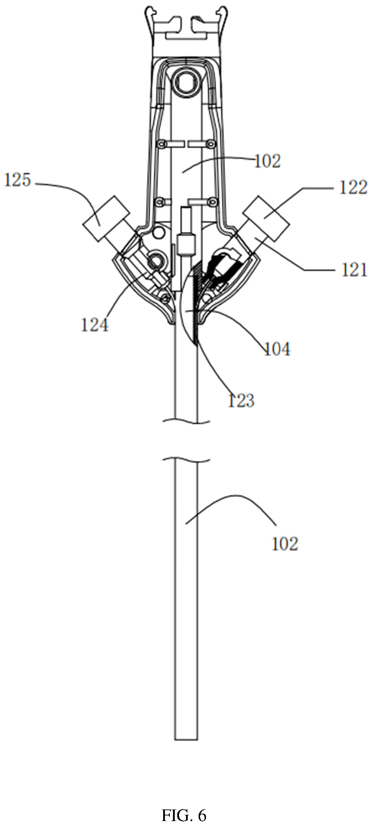

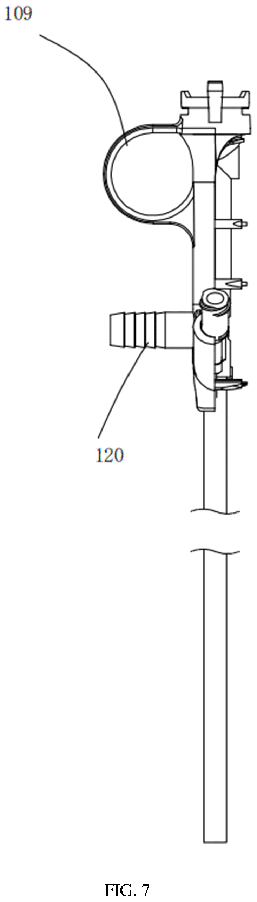

As shown in FIGS. 2-13, the pressure measuring sheath 10 provided in the embodiment comprises a handle and an elongated sheath tube 102. The handle is used as a handle grasped by the hand of an adult user. The user holds the handle with one hand to flexibly operate the pressure measuring sheath 10. The size and shape of the sheath tube 102 are suitable for entering a target area in the body such as the renal pelvis through the body lumen, and extend longitudinally from a proximal end to a distal end. The handle is connected to the proximal side of the sheath tube 102 so that the handle can be kept outside the body.

The sheath tube 102 defines at least a first lumen extending longitudinally from a proximal end to a distal end, and the handle comprises a handle body 101 and a first connecting member 120 arranged on the handle body 101, in which the handle body 101 extends in a direction consistent with the axis of the sheath 102 for easy gripping by the user.

The handle body 101 defines a port 108 communicated with the first lumen 104, and a seal 106 is arranged at the port 108. The seal 106 comprises a passage therethrough so that after the medical equipment passes through the passage, a sealed cavity is formed between the first lumen 104 and the body.

The first connecting member 120 has a first channel, the first channel is communicated with the first lumen to form a drainage path, and the first connecting member 120 is suitable for connecting with a negative pressure device so that liquid medium in the body can be discharged from the body through the drainage path. Exemplarily, the first connecting member 120 can be a Luer connector, etc. The Luer connector is connected to the suction device, and the waste liquid enters the collection container of the suction device after passing through the first lumen 104 and the first connecting member 120.

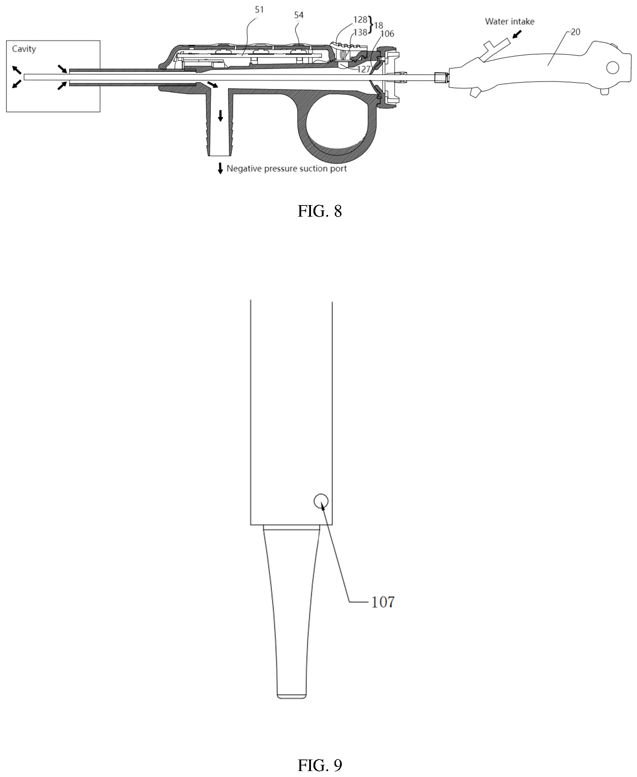

As shown in FIG. 8, the handle body 101 is further provided with a pressure regulating member 18, and the pressure regulating member 18 is used to adjust the pressure of the first lumen. In this way, when the suction pressure is too high and the organ in the renal pelvis is sucked flat due to excessive pressure, air can be quickly introduced through the pressure regulating member 18 for rapid pressure relief to ensure the progress of the operation. In some preferred embodiments of the present disclosure, the pressure regulating member 18 is in fluid communication with the first lumen and is set away from the drainage path, that is, the pressure regulating member 18 is far away from the discharge path of the waste liquid. In this way, when the pressure regulating member 18 is manually operated, it will not be contaminated with liquid, thereby achieving contactless, i.e., aseptic operation. In addition, the cleverness of arranging the pressure regulating member 18 on the handle body 101 is that: When the suction pressure is too high and causes failure to suck stones, the user needs to manually increase the suction force. Based on the pressure regulating member 18 provided in this embodiment, holding the handle body 101, by repeatedly and quickly controlling the state of the pressure regulating member 18, the pressure in the cavity will fluctuate rapidly between pressure relief and negative pressure. Repeated pressure fluctuations can produce a strong suction effect, making it easier for objects such as adsorbed or stuck stones to be extracted. Rapidly changing pressure can generate enough impact force to help clear blockages and ensure the smooth flow of suction channels. By controlling pressure fluctuations, stronger suction can be provided when needed, improving the efficiency and success rate of surgery.

In some embodiments of the present disclosure, the pressure regulating member 18 comprises a relief valve, a relief cavity 127 communicated with the first lumen 104 of the sheath tube 102 is provided on the handle body, and a relief valve for sealing or opening the relief cavity 127 is provided on the handle body 101. In some preferred embodiments of the present disclosure, the relief cavity 127 is arranged to the proximal side of the liquid outlet, that is, the path of the relief cavity 127 is not used as a discharge path for waste liquid, and the relief cavity is far away from the discharge path of the waste liquid. In this way, the waste liquid does not pass through the relief cavity 127, and when the relief valve is manually operated, it will not be contaminated with liquid , thereby achieving contactless, i.e., aseptic operation. In addition, the cleverness of arranging a relief cavity 127 and a relief valve on the handle body 101 is that: When the suction pressure is too high and causes failure to suck stones, the user needs to manually increase the suction force. Based on the relief cavity 127 and the relief valve provided in this embodiment, holding the handle body 101, when the relief valve is quickly opened, outside air rapidly enters the cavity, causing the pressure to rise quickly and thus achieving the pressure relief effect; and when the relief valve is quickly closed, the suction device continues to operate, and the intracavity pressure drops rapidly to form a negative pressure for suction. By repeatedly and quickly opening and closing the relief valve, the pressure in the cavity will fluctuate rapidly between pressure relief and negative pressure. Repeated pressure fluctuations can produce a strong suction effect, making it easier for objects such as adsorbed or stuck stones to be extracted. Exemplarily, the relief valve comprises a pressing part 128, the pressing part is connected to a relief hose (relief cavity 127), and an openable and closable valve 138 is formed on the relief hose (relief cavity 127), in this way, a user can control the opening and closing of the valve 138 by the force with which the pressing part 128 is pressed. Exemplarily, the pressing part is connected to the relief pipe, and pressing the pressing part can adjust the opening and closing of the valve thereof, thereby adjusting the volume of outside air entering the first lumen.

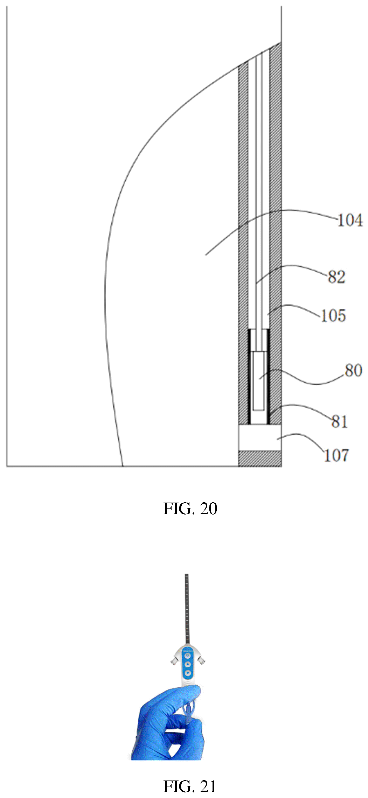

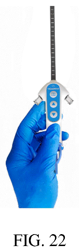

In one embodiment of the present disclosure, the introducer sheath further comprises a PCB 51 and an operating button 54. The PCB 51 is arranged in the handle body. At least one data connector 52 is provided on the PCB 51. The data connector 52 extends from the handle body 101. The data connector 52 is suitable for connecting to external perfusion and suction equipment. Further, the handle body 101 is provided with an operating button 54. The user controls the operating button 54 to realize the control operation of perfusion and suction; and specifically, the operating button 54 is located at the upper part of the handle body 101 for controlling the operation part of injection and suction, and the pressure regulating member 18 is also arranged at the upper part. In this way, as shown in FIGS. 21-22, the upper part of the handle body 101 forms an operation area, and operations such as injection, suction, pressure relief and increasing suction force are realized on the handle. All these operations can be done with your fingers, which greatly simplifies the use process. The operating button 54 and the pressure regulating member 18 are both arranged at the upper part, and there is no need to frequently move the handle during operation. You only need to operate your fingers on the upper part of the handle body 101. This reduces the range of hand movement and improves operational efficiency. Compared with the traditional design that requires controlling multiple devices at the same time, the present disclosure integrates multiple control functions into one. Doctors no longer need to switch or coordinate multiple devices, but can perform multiple functions with just one handle, which reduces the complexity of operation.

The PCB 51 is arranged in the middle position of the handle body 101, which optimizes the internal space layout and makes the shape and weight distribution of the handle body 101 more reasonable. The handle body 101 is designed to be more ergonomic and increase the comfort of use. In some examples of the present disclosure, a finger ring 109 is provided at the bottom of the handle body 101, in this way, a finger ring 109 is provided at the bottom of the handle body 101, and the operating surface is located on the top surface of the handle body 101. This design is ergonomic and ensures that the user can maintain a natural and comfortable holding posture during operation. The finger ring 109 design provides additional support points to help users hold the handle more stably and reduce fatigue of hand muscles. Due to the presence of the finger ring 109, the user can pass one finger through the finger ring 109, so that the handle can be firmly held and other fingers can be flexibly used for operation. The operating surface on the top of the handle body 101 is more accessible to fingers, and the operation is simpler and faster. The arrangement of the finger ring 109 makes the center of gravity of the handle more stable, and the weight can be dispersed more evenly when holding the handle, which improves the comfort of operation. The finger ring 109 makes the handle more stable, and the handle is not easy to slide or rotate during operation, thereby improving the accuracy and control of the operation. Based on the above improvements, the design of the finger ring 109 makes the handle body 101 more in line with the user's usage habits and needs, especially in scenarios that require long-term holding and fine operation, which significantly improves the user experience.

In an embodiment of the present disclosure, a liquid outlet is provided at the bottom end of the sheath tube 102, where the bottom end refers to the end facing the ground, that is, the design of the bottom liquid outlet uses gravity to make waste liquid and stones flow downward, thereby enhancing the discharge efficiency. One end of the first connecting member 120 is arranged at the liquid outlet, and the other end extends out of the handle body 101. The axis of the first channel defined by the first connecting member is perpendicular to the axis of the sheath tube 102. In this way, compared with the inclined side arm led out from the sheath tube 102 for waste liquid discharge, the present disclosure forms a straight discharge path for waste liquid discharge, which flows directly into the first connecting member 120 through the bottom end of the sheath tube 102. This straight path reduces the resistance encountered by liquid and stones in the channel and reduces the risk of blockage. Since the first connecting member 120 is directly connected to the liquid outlet, there is no bent pipe, and the waste liquid and stones do not need to change the flow direction when discharged. This design significantly reduces the chance of stones being trapped and stuck in the pipe. In addition, by arranging the first connecting member below the handle body 101 rather than on the side, the doctor's operation is not interfered with by the negative pressure suction device, further improving the fluency and accuracy of the operation.

In this embodiment, a first connecting part 132 suitable for detachable connection with an expander 60 is arranged at the port 108 of the handle body 101, and the expander 60 has a hand-held part and an elongated catheter. The first connecting part 132 is suitable for detachable connection with a second connecting part 601 of the hand-held part of the expander 60. Specifically, the first connecting part 132 is snap-connected to the second connecting part 601. The rubber seal 106 is connected to the elongated sheath tube, the rubber seal is located in the handle body 101, and the rubber seal is connected to the proximal end of the sheath tube. The rubber seal is used for sealing and penetrating the elongated expander 60 or the endoscope 20 catheter. Specifically, a pressing piece is provided at the proximal end of the handle body 101. The pressing piece comprises a pressing part 133 pressed above the rubber valve and a rotating part 134 that rotates with the handle body 101. The rubber seal is provided with a lower pressure relief hole 130, and the pressing cover is provided with an upper pressure relief hole 130. The pressing cover is driven to rotate by the rotation of the rotating ring so that the lower pressure relief hole 130 is communicated with the upper pressure relief hole 130 to achieve pressure stabilization.

In addition, the proximal end of the handle body 101 is also provided with a limiting part 135 to prevent the expander 60 from coming out, so as to limit the rotation and disengagement of the expander 60 during use in conjunction with the sheath, thereby facilitating operation and improving efficiency.

In one example of the present disclosure, the distal end of the sheath tube 102 is a flexible section 136 that can be bent. The endoscope 20 comprises an operating part and a bending part. The operating part can drive the bending part to bend. The endoscope 20 is inserted into the sheath tube 102. The bending part is accommodated in the flexible section 136. The bending part can drive the flexible section 136 to bend.

### Embodiment 2

Based on Embodiment 1, the present disclosure further provides a pressure measuring sheath capable of detecting pressure in the body, such as in the renal pelvis. A pressure measuring lumen 105 extending longitudinally from a proximal end to a distal end is formed in the wall of the sheath tube, in which the pressure measuring lumen 105 is separated and arranged side by side with the first lumen 104, and a pressure measuring port for sensing the liquid pressure in the body is provided at the distal side of the pressure measuring lumen 105, and the handle comprises a second connecting member 124 arranged on the handle body. The second connecting member 124 has a second channel communicated with the pressure measuring lumen 105, and a pressure sensor is provided on the pressure measuring lumen 105, the second channel or any path connected thereto, which can collect pressure data of the pressure measuring lumen. Exemplarily, the pressure sensor 50 can be arranged at the distal end of the endoscope 20, or at the distal end of the pressure measuring sheath 10, or at the pressure measuring lumen 105 in the pressure measuring sheath 10, or at any position on the pressure measuring path communicated with the pressure measuring lumen 105, such as an external device, where the position comprises but is not limited to the second connecting member 124 communicated with the pressure measuring lumen 105.

Further, in this embodiment, the pressure sensor 50 is installed on the inner side or outer side of the second channel. In this embodiment, the outer wall of the second channel is provided with a pressure measuring hole 126, and the pressure sensor 50 is arranged on the inner side of the handle body and at least its sensing part is arranged at the pressure measuring hole 126 to sense the pressure of the pressure measuring lumen 105. It occupies almost no space in the second channel for installation, and the second channel 124 can also be used as another auxiliary lumen to avoid occupying resources with other instruments. It can be understood that the detection signal of the pressure sensor 50 can be connected to an external device via a connecting wire for transmission, processing, and other operations.

In some embodiments of the present disclosure, the pressure sensor 50 is a gauge pressure sensor, the sheath tube 102 is connected to the handle body 101 in a sealed manner to construct a calibration cavity 103 in the handle body, and the calibration cavity 103 is connected to the outside world so as to calibrate and adjust the pressure sensor 50; a PCB 51 and a pressure sensor 50 are installed in the calibration cavity 103, a pressure measuring hole 126 communicated with the calibration cavity is provided on the outer wall of the second channel, the gauge pressure sensor is positioned in the cavity 103 and at least a sensing film thereof is arranged at the pressure measuring hole 126 to collect pressure in the pressure measuring lumen, and the second channel has an opening that can be connected to the outside world; the opening can be opened or closed by a sealing part such as a second sealing cap 125, and the calibration of the pressure sensor 50 is achieved by cooperating with the calibration cavity 103 through the opening.

Specifically, the working principle of the gauge pressure sensor is to determine the pressure value by measuring the pressure change of fluid or gas on the sensing membrane of the sensor, and then compare this value with the ambient pressure to obtain the relative pressure value. If the pressure at the measuring point is higher than the atmospheric pressure, the sensor outputs a positive value; if it is lower than the atmospheric pressure, it outputs a negative value or zero value. When the pressure measuring sheath does not enter the body, the pressure measuring port 107 at the distal end of the pressure measuring sheath 10 is connected to the calibration cavity 103 to achieve calibration of the gauge pressure sensor. When the pressure measuring sheath enters the body, the second sealing cap 125 is opened, and the second channel is connected to the outside world. At this time, it cooperates with the calibration cavity 103 to achieve calibration of the gauge pressure sensor. In addition, the gauge pressure sensor is installed in the handle body 101. Compared with being installed on an external setting such as a host controller, it is arranged at the handle position to reduce the volume of the piezometric cavity, respond quickly and accurately to pressure changes, avoid signal attenuation and hysteresis in a large space, and have higher accuracy.

In the present embodiment, the pressure sensor 50 is located in the main body of the handle body 101 so as to be electrically connected to the PCB 51, and the data connector 52 is used to connect to external devices such as a host controller, a perfusion device, a suction device, and a display device to realize the transmission of pressure signals and the control of perfusion and suction operation states.

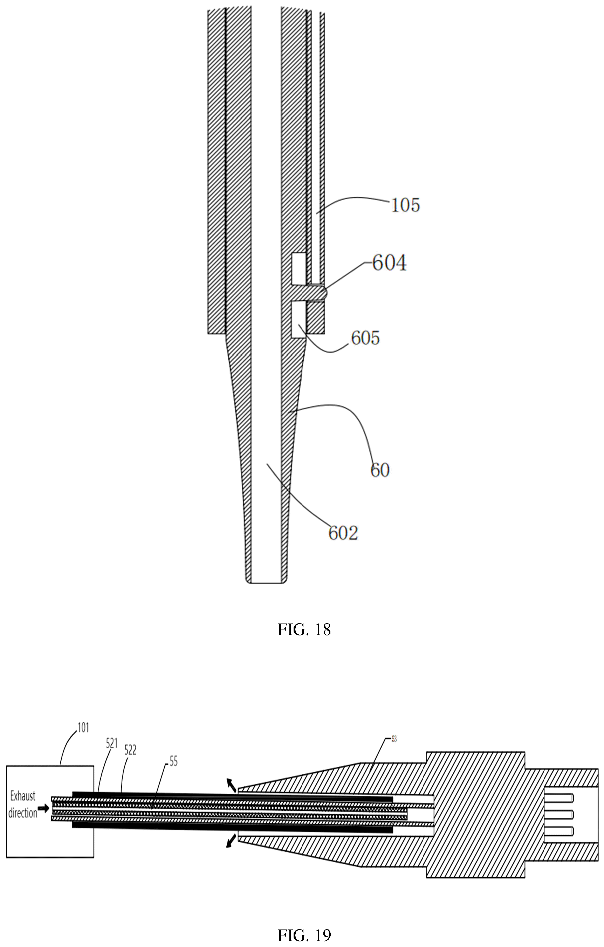

As shown in FIG. 19, the data connector 52 (including the wire core 521 and the insulation sheath 522) has an exhaust pipe 55 that communicates with the outside world with the handle body 101 to achieve conduction between the calibration cavity in the handle body and the outside world. In addition, due to the pressing operation of the operating button or other operations on the handle, the pressure change in the sealed calibration cavity 103 in the handle body 101 will cause the sensor to be basically offset. The design of the exhaust pipe eliminates the pressure fluctuation caused by the operating button 54, maintains the pressure stability in the handle body 101, and avoids the reduction of the pressure measurement precision of the pressure sensor 50 caused by the pressure fluctuation in the handle body 101. By rationally arranging the pressure sensor 50 and its ancillary equipment, combined with the design of the sealed cavity and the exhaust pipe 55, accurate pressure monitoring of the pressure measuring lumen 105 is achieved while ensuring system stability and ease of operation.

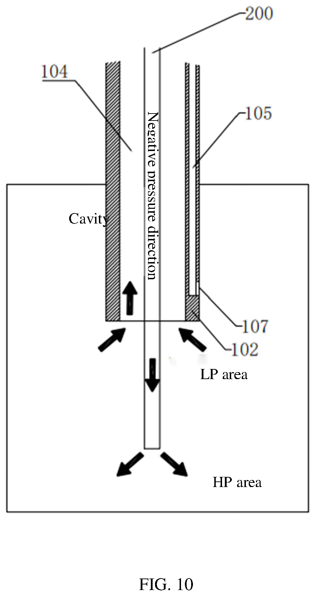

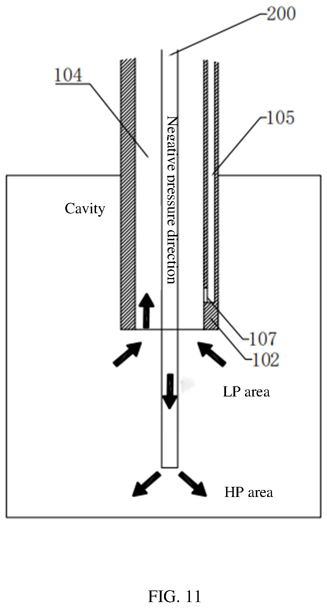

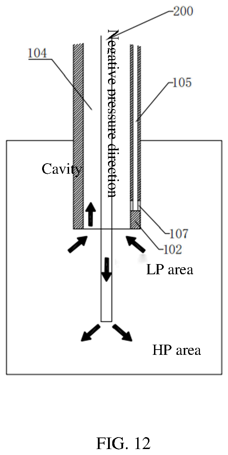

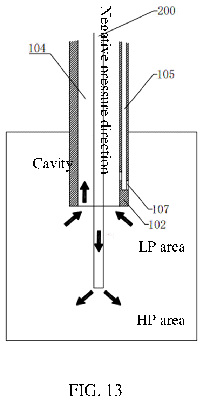

As mentioned above, the distal end of the pressure measuring lumen 105 has a pressure measuring port 107, and the pressure measuring port 107 is suitable for sensing intracavity pressure in the human body and transmitting it to the pressure measuring lumen. There are many ways to arrange the pressure measuring port: exemplarily, as shown in FIG. 10, the pressure measuring port 107 is communicated with the outside world but not communicated with the first lumen 104, that is, the outer wall of the pressure measuring lumen 105 is opened to form a pressure measuring port. This external pressure measuring channel port is communicated with the high-pressure area in the cavity, providing high measurement accuracy and truly reflecting intracavity pressure. However, foreign matter can easily enter the pressure measuring lumen through the pressure measuring port at the outer side, and the pressure measurement precision decreases after the pressure measuring lumen is blocked; in another embodiment, as shown in FIG. 11, the pressure measuring port 107 is communicated with the first lumen 104 but not communicated with the outside world, that is, the inner wall of the pressure measuring lumen 105 is opened to form a pressure measuring port, and the pressure measuring port is communicated with the low-pressure area in the cavity. The measured value will be lower than the overall pressure in the cavity, but the risk of foreign matter blocking the pressure measuring lumen is eliminated; and in further another embodiment, as shown in FIGS. 12 and 13, the pressure measuring port 107 is communicated with the outside world and the first lumen 104. At this moment, both the inner wall and the outer wall of the pressure measuring lumen 105 have openings. In this embodiment, the opening of the inner wall corresponds radially to the opening of the outer wall. The full-through pressure measuring channel port is simultaneously communicated with both the high-pressure area and the low-pressure area in the cavity, providing high measurement accuracy and truly reflecting intracavity pressure, but there is also a risk of tube blockage. Or the opening of the inner wall is staggered with the opening of the outer wall, for example, it is Z-shaped. The Z-shaped pressure measuring port is simultaneously communicated with both the high-pressure area and the low-pressure area in the cavity, providing high measurement accuracy and truly reflecting intracavity pressure, with no risk of tube blockage. More preferably, the distal end of the pressure measuring lumen 105 is located proximal to the proximal side of the first lumen, and a pressure measuring port penetrating the outside world and the first lumen is provided at the distal end of the pressure measuring lumen. That is, the distal end of the pressure measuring lumen is not consistent with the distal end of the first lumen, and the pressure measuring port penetrates both the outside world and the first lumen.

In some embodiments of the present disclosure, a pressure relief hole 130 is provided at the proximal end of the sheath tube 102 provided by the present disclosure. The pressure relief hole 130 is suitable for communicating with the outside world to facilitate air entry. When the sheath tube 102 is inserted through an instrument or the like, it will cause pressure fluctuations. By opening the pressure relief hole 130 and introducing an appropriate amount of air to ensure the stability of intracavity pressure, in this way, the pressure fluctuation caused by external operation is avoided and the pressure measurement precision is improved. It can be understood that the pressure relief hole 130 should be a small hole or a hole with valve control to introduce an appropriate amount of air when necessary to relieve excessive internal pressure. In an embodiment of the present disclosure, a seal 106 is provided at the proximal end of the sheath tube102, and the endoscope 20 or the expander 60 is sealed and inserted into the sheath tube 102. The seal 106 is provided with a pressure relief hole 130, and the pressure relief hole 130 is provided with a switch to open or close the pressure relief hole 130, thereby solving the influence of pressure fluctuations caused by instrument entry on pressure detection and achieving small-scale pressure relief. Optionally, the sheath tube 102 comprises a seal 106 and an elongated sheath tube. The seal 106 has a valve port. The seal 106 is connected to the proximal end of the sheath tube. The valve port is used for sealing and penetrating the elongated expander 60 or the endoscope 20. A pressing piece is provided at the proximal end of the handle body 101. The pressing piece comprises a pressing part 133 pressed above the seal 106 and a rotating part 134 that rotates with the handle body 101. The seal 106 is provided with a lower pressure relief hole 130, and the pressing part 133 is provided with an upper pressure relief hole 130. The pressing part 133 is driven to rotate by the rotation of the rotating ring so that the lower pressure relief hole 130 is communicated with the upper pressure relief hole 130 to achieve pressure stabilization.

### Embodiment 3

In some embodiments of the present disclosure, the present disclosure utilizes dual-channel perfusion, and the flow of fluid between different channels is affected by the pressure difference. Fluid in high-pressure areas will flow toward low-pressure areas, and low-pressure areas will attract more fluid. The endoscope 20 enters the sheath tube 102, and the waste liquid is sucked out of the body through the first lumen 104 of the sheath tube 102. The liquid enters the body through the catheter of the endoscope 20. When the negative pressure suction pressure in the sheath tube 102 is very high, the pressure inside the sheath tube 102 is much lower than that in the endoscope 20. Such a huge pressure difference will cause more liquid to enter the body, which will lead to excessive perfusion of the liquid. Excessive injection pressure is formed in local areas, making it difficult to pump waste liquid such as stones.

Based on this, the handle body 101 provided in this present disclosure is provided with a third connecting member 121, and the third connecting member 121 can be blocked by a first sealing cap 122. When liquid delivery is required, the first sealing cap 122 is opened, and when it is not needed, the first sealing cap 122 is closed to avoid the influence of negative pressure. The sheath tube 102 has an auxiliary lumen 123 isolated from the first lumen 104 and the pressure measuring lumen 105, the third connecting member 121 is communicated with the auxiliary lumen 123, and the third connecting member 121 and the auxiliary lumen 123 form a liquid inlet channel for the perfusion liquid medium to enter. In this way, the perfusion solution enters the body through the third connecting member 121 and the auxiliary lumen 123 to generate slight pressure fluctuations. In this way, a dynamic pressure environment is formed around the stone, which promotes the loosening and movement of the stone. Pressure fluctuations will periodically change the flow rate and direction of the liquid, thereby generating impact force on the stone, gradually reducing the adhesion and static friction between the stone and the surrounding tissue. In addition, the introduction of the auxiliary lumen 123 provides more liquid inlet paths, ensures a stable supply of liquid medium, avoids pressure instability caused by uneven liquid supply from a single channel, and solves the technical problem in the present disclosure that stones caused by excessive perfusion pressure are difficult to be effectively aspirated.

Optionally, the inner diameters of the second channel 124 and the third connecting member 121 are larger than the inner diameters of the pressure measuring lumen 105 and the auxiliary lumen 123, in this way, facilitating the entry of external instruments.

### Embodiment 4

A water film is easily formed at the pressure measuring port 107 at the distal end of the pressure measuring lumen 105. The formation of this water film makes it difficult for the pressure signal to be transmitted to the pressure sensor 50 in a timely and accurate manner, making it difficult to detect the pressure in real time. In addition, the water film tension causes a signal transmitted to the pressure sensor 50 to deviate from an actual pressure signal, thereby affecting the accuracy of measurement. Exemplarily, the pressure sensor 50 has a measured pressure P=P3=P1+P2, where P1 is intracavity pressure, P2 is water film tension, and P3 is pressure in the pressure measuring lumen 105.

Based on this, in some embodiments of the present disclosure, the pressure measuring sheath 10 also comprises a tension elimination mechanism to eliminate the influence of water film on intracavitary pressure detection.

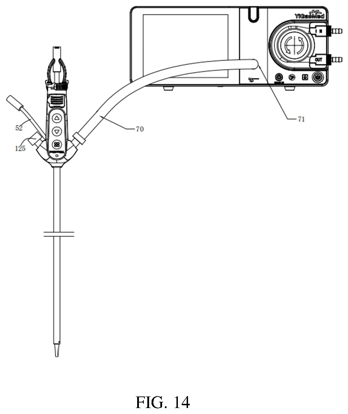

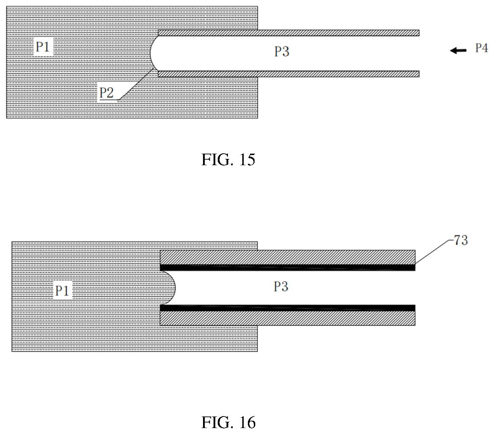

As mentioned above, as shown in FIG. 14, the handle body 101 is provided with a first connecting member 124 communicated with the pressure measuring lumen 105. The first connecting member 124 has an opening, which can be sealed or opened by the second sealing cap 125. The first connecting member 124 forms an air intake pipe for high-frequency and low-pressure gas from the outside world to enter to eliminate the water film; since the pressure sensor 50 does not occupy the space of the first connecting member 124, the first connecting member 124 can be used as other auxiliary lumen, thereby improving the space utilization efficiency of the pressure measuring sheath 10. The interference of the water film on the signal transmission of the pressure sensor 50 is effectively eliminated, ensuring timely and accurate transmission of the pressure signal. Exemplarily, as shown in FIG. 15, the second sealing cap 125 of the second channel 124 is opened, and a high-frequency micro-pressure gas such as carbon dioxide is delivered to the second channel 124 to break a water film tension balance formed by the liquid in the cavity at the distal port 108 of the pressure measuring lumen 105; at this moment, the pressure measuring module measures pressure P=P3+P4=P1+P2 (when the water film tension balance is broken, P4=P2, and P=P3=P1), in which P1 is intracavity pressure, P2 is water film tension, P3 is pressure in the pressure measuring lumen 105, and P4 is the high-frequency pulse carbon dioxide gas pressure P4. Exemplarily, the second connecting member 124 is connected to the carbon dioxide air pump 71 interface through a connecting pipe 70.

In addition, a one-way valve 74 is arranged at the second connecting member 124 to allow only gas to be pumped into the pressure measuring lumen 105 from the outside world to eliminate the drop in pressure accuracy caused by the water film tension. If the one-way valve 74 is not arranged, after the second sealing cap 125 is blocked, foreign matter and water column will re-enter the pressure measuring lumen 105. When the second sealing cap 125 is closed, air will be pressed in, causing a pressure difference in the pressure measuring lumen 105. Combined with the influence of the water film, the pressure measurement precision decreases. The use of the one-way valve 74 can prevent the water in the cavity from retrogradely entering the pressure measuring lumen 105 after the inflation is completed, thereby improving the pressure measurement precision.

In some other embodiments, as shown in FIG. 16, the inner wall of the pressure measuring lumen 105 is provided with a hydrophilic or hydrophobic coating 73, the water film tension can be regarded as non-contacting to the pressure measuring lumen 105, and at this moment, the pressure measuring module measures pressure P=P3=P1, The retention of water film is reduced, and the self-cleaning ability and measurement accuracy of the pressure measuring lumen 105 are improved.

This embodiment effectively eliminates the influence of water film on pressure detection by introducing high-frequency and low-pressure gas, coating treatment, one-way valve and other measures, significantly improving the measurement accuracy and system stability.

### Embodiment 5

When the pressure measuring sheath 10 (usually used for surgical operations such as catheterization) enters the body cavity, negative pressure suction and perfusion have not yet begun. At this time, the expander 60 has been inserted into the pressure measuring sheath 10 to ensure that the pressure measuring sheath 10 can smoothly enter the body cavity. Since the expander 60 occupies the first lumen 104 of the sheath tube 102, it is easy for foreign matter to enter the pressure measuring lumen 105, causing the pressure measuring lumen 105 to be blocked and the pressure measurement precision to drop sharply.

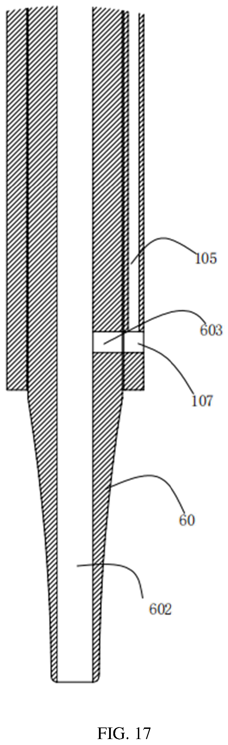

The expander 60 is provided with a blocking part that limits foreign matter from entering the pressure measuring lumen 105 through the pressure measuring port 107, as shown in FIG. 17.

In some embodiments of the present disclosure, the pressure measuring port 107 is communicated with both the outside world and the first lumen. At this time, the inner wall and the outer wall of the pressure measuring lumen have openings. The opening of the inner wall corresponds radially to the opening of the outer wall, that is, the distal end of the pressure measuring lumen is inconsistent with the distal end of the first lumen, and the pressure measuring port penetrates the outside world and the first lumen. A cavity is formed inside the expander 60, and the side wall of the cavity has a side hole 603 communicated with the pressure measuring port 107 at the distal end of the pressure measuring lumen 105. Foreign matter can enter from the side hole 603 of the expander 60 and be discharged from the cavity.

The side hole 603 is not less than the diameter of the pressure measuring port 107. The diameter of the side hole 603 of the expander 60 is not less than the diameter of the pressure measuring port 107, ensuring that foreign matter can smoothly pass through the side hole 603 and be discharged from the cavity, preventing the pressure measuring lumen 105 from being blocked, and improving the safety and convenience of surgical operations.

In some other embodiments, as shown in FIG. 18, the expander 60 is provided with a plugging part 60 that can block the pressure measuring port 107 at the distal end of the pressure measuring lumen 105 to limit foreign matter from entering the pressure measuring lumen 105. In this way, when entering the body cavity, the expander 60 is pulled out of the sheath tube 102, avoiding the introduction of foreign matter into the pressure measuring lumen 105, ensuring the patency and pressure measurement precision of the pressure measuring lumen 105. Exemplarily, the plugging part 60 is made of a flexible material to allow the expander 60 to be withdrawn from the sheath tube 102. The plugging part 60 is made of a flexible material, allowing the expander 60 to be smoothly withdrawn from the sheath tube 102 during use, thus avoiding operational inconvenience and potential damage that could be caused by hard materials.

A gap is reserved between the plugging part 60 and the pressure measuring port 107. An appropriate gap is reserved between the plugging part 60 and the pressure measuring port 107 to ensure the sealing effect while avoiding operational difficulties caused by excessive tightness and pressure changes in the pressure measuring lumen 105. A groove 605 is formed on the outer wall of the expander in the area adjacent to the plugging part, which allows the plugging part to retract into the groove after withdrawing from the pressure measuring port, thereby facilitating the smooth withdrawal of the expander from the sheath tube.

By arranging a blocking part and an plugging part 60 on the expander 60, the issue of foreign matter entering the pressure measuring lumen 105 during entry of the pressure measuring sheath 10 into the body cavity-leading to blockage and reduced pressure measurement precision-is resolved.

In this embodiment, as shown in FIG. 20, a pressure sensor 50 is provided at a position near the pressure measuring port of the pressure measuring lumen 105. A signal line 82 is arranged in the pressure measuring lumen to transmit pressure signals. The pressure sensor 50 is a resistance bridge sensor 80, and the sensor is placed at the distal end of the sheath. The temperature acts on the sensor surface, and the resistance of the temperature measuring bridge arm changes to generate a pressure difference. At the same time, the gas and liquid pressures act on the sensor surface. The sensor generates micro-strain, and the resistance of the pressure measuring bridge arm changes to generate a voltage difference. Then the two sets of voltage signals are converted into digital signals through the internal collector, and finally the data is read out through the SPI interface. In this way, the resistance bridge sensor can measure both temperature and pressure, and realize accurate data collection and transmission through the principle of resistance change and pressure difference.

A protective sleeve is provided in the body cavity at the distal side of the pressure measuring lumen 105, and the resistance bridge sensor 80 is installed in the protective sleeve. The sensing surface of the resistance bridge sensor 80 is communicated with the pressure measuring port 107 at the distal end of the pressure measuring lumen 105. It is preferably protected by a stainless steel protective sleeve to prevent the sensor from being accidentally squeezed or accidentally hit by energy instruments such as lasers.

The resistance bridge sensor 80 transmits signals through a signal line 82 arranged in the pressure measuring lumen.

In the description of the specification, descriptions with reference to the terms "one embodiment", "some embodiments", "illustrative embodiment", "example", "specific example", or "some examples" mean that specific features, structures, materials or characteristics described in combination with the embodiment or example are contained in at least one embodiment or example of the present disclosure. In the specification, the schematic expressions of the above terms do not necessarily refer to the same embodiments or examples. Moreover, the specific features, structures, materials, or characteristics described may be combined in any one or more embodiments or examples in a suitable manner.

Although embodiments of the present disclosure have been shown and described, the person of ordinary skill in the art can understand that various changes, modifications, substitutions and variations may be made to these embodiments without departing from the principle and purpose of the present disclosure, and the scope of the present disclosure is defined by the claims and their equivalents.

## Claims

1. An introducer sheath, comprising
a sheath tube, having a size and a shape suitable for passing through the lumen of a body into a target area in the body and extending longitudinally from a proximal end to a distal end, and the sheath tube defining at least a first lumen extending longitudinally from the proximal end to the distal end;
a handle, being connected to a proximal side of the sheath tube so that the handle can be kept outside the body, wherein the handle comprises a handle body and a first connecting member and a pressure regulating member arranged on the handle body; the handle body extends in a direction consistent with the axis of the sheath tube for easy holding by a user; the handle body defines a port communicated with a first lumen so that medical equipment can enter the first lumen through the port; the first connecting member has a first channel, the first channel is communicated with the first lumen to form a drainage path, the first connecting member is suitable for connecting with a negative pressure device so that liquid medium in the body can be discharged from the body through the drainage path, and the pressure regulating member is in fluid communication with the first lumen and is arranged away from the drainage path so as not to contact the liquid when adjusting pressure of the drainage path; and
a seal, being arranged at the port, where the seal comprises a passage passing therethrough so that after the medical equipment passes through the passage, a sealed cavity is formed between the first lumen and the body.

2. The introducer sheath according to claim 1, wherein the introducer sheath further comprises
a printed circuit board (PCB), being mounted in the handle body, where the PCB is provided with at least one data connector, the data connector is configured to be suitable for connecting an external perfusion and suction device to transmit signals; and
an operating button, being arranged on the handle body and connected to the PCB to control operating states of the perfusion and suction device.

3. The introducer sheath according to claim 2, wherein a pressure measuring lumen extending longitudinally from a proximal end to a distal end is formed in the wall of the sheath tube, a pressure measuring port for sensing the liquid pressure in the body is provided at the distal side of the pressure measuring lumen, a pressure sensor is provided on the pressure measuring lumen or any path connected thereto, and the pressure sensor is connected to the PCB; preferably, the handle comprises a second connecting member arranged on the handle body, the second connecting member has a second channel communicated with the pressure measuring lumen, and the pressure sensor is arranged at the second channel to sense the pressure in the second channel;
preferably, the sheath tube is connected to the handle body in a sealed manner to construct a calibration cavity in the handle body, and the calibration cavity is connected to the outside world; a PCB and a pressure sensor are installed in the calibration cavity, the pressure sensor is a gauge pressure sensor, a pressure measuring hole communicated with the calibration cavity is provided on the outer wall of the second channel, the gauge pressure sensor is positioned in the cavity and at least a sensing film thereof is arranged at the pressure measuring hole to collect pressure in the pressure measuring lumen, and the second channel has an opening that can be connected to the outside world; the opening can be opened or closed by a sealing part, and the calibration of the pressure sensor is achieved by cooperating with the calibration cavity through the opening;
preferably, the second connecting member is configured to be suitable for connection with an external air intake device to eliminate water film tension of the pressure measuring lumen;
optionally, the pressure measuring port is communicated with the outside world and/or the first lumen;
preferably, the distal end of the pressure measuring lumen is located at the proximal side to the distal end of the first lumen, and the distal end of the pressure measuring lumen is provided with a pressure measuring port that penetrates the outside world and the first lumen;
optionally, a pressure sensor is provided at the distal side of the pressure measuring lumen, and a signal line is arranged in the pressure measuring lumen to transmit pressure signals collected by the pressure sensor to the PCB;
optionally, the surface of at least the cavity at the distal side of the pressure measuring lumen is coated with a hydrophilic or hydrophobic coating;
optionally, a one-way valve is provided on the second connecting member to allow only gas to enter the pressure measuring lumen from the outside world in one direction.

4. The introducer sheath according to claim 1, wherein a liquid outlet is provided at the bottom end of the first lumen, the first connecting member is located below the handle body, and the axis of the first channel is perpendicular to the axis of the first lumen to form a linear drainage path.

5. The introducer sheath according to claim 1, wherein the pressure regulating member comprises a pressure regulating valve communicated with the first channel, and the first pressure regulating valve is arranged on the proximal side of the first connecting member.

6. The introducer sheath according to claim 3, wherein a hand-held positioning portion is provided at the bottom of the handle body, the first connecting member is arranged at the bottom of the handle body, the second connecting member is arranged on a side wall of the handle, and the pressure regulating member and the operating button are located at the upper part of the handle.

7. The introducer sheath according to claim 1, wherein the handle comprises a third connecting member arranged on the handle body, the sheath tube defines an auxiliary lumen extending from the proximal end toward the distal end, and the third connecting member is communicated with the auxiliary lumen to form a fluid replenishment channel.

8. The introducer sheath according to claim 1, wherein the pressure regulating member comprises a relief valve arranged on the handle body, the handle body is provided with a relief cavity communicated with the first lumen, and the relief valve seals or opens the relief cavity; preferably, the relief valve comprises a pressing part, the pressing part is connected to a relief hose, and an openable and closable valve is formed on the relief hose..

9. The introducer sheath according to claim 1, wherein an exhaust pipe communicating with the outside world and the calibration cavity is provided in the data connector to maintain pressure stability in the handle body;
optionally, the seal is connected to the proximal end of the sheath tube, a pressing piece is provided at the proximal end of the handle body, and the pressing piece comprises a pressing part pressed above the seal and a rotating part that rotates with the handle body; the seal is provided with a lower pressure relief hole, and the pressing part is provided with an upper pressure relief hole; the pressing part is driven to rotate by the rotation of the rotating part so that the lower pressure relief hole is communicated with the upper pressure relief hole to achieve pressure stabilization.

10. A perfusion and suction system, comprising
the introducer sheath according to any one of claims 1-9,
an endoscope, being sealed through a port of the handle body into the first lumen, and waste liquid being discharged from a gap between the first lumen and the endoscope;
a perfusion device, being connected to an endoscope to deliver a liquid medium into a human body through an endoscopic catheter;
a suction device, being connected to the first connecting member to generate negative pressure in a drainage path so as to suck out waste liquid containing stones from the human body; and
a host controller, the host controller being connected to the perfusion device, the suction device, and the PCB to control an operating status of perfusion and suction.
